# EUROPEAN PATENT SPECIFICATION

(11) **EP 1 480 949 B1**
(45) Date of publication and mention of the grant of the patent: **29.04.2015**
(21) Application number: 03711292.7
(22) Date of filing: 28.02.2003
(51) Int. Cl.: C07C 405/00, A61K 31/557

(54) **PREPARATION OF PROSTAMIDES**
ZUBEREITUNG VON PROSTAMIDE
PREPARATION DE PROSTAMIDES

(30) Priority: 01.03.2002 US 360847 P
(43) Date of publication of application: 01.12.2004
(73) Proprietor: ALLERGAN, INC., Irvine, CA 92612 (US)
(72) Inventor: GARST, Michael, E., Newport Beach, CA 92660 (US); OUDENES, Jan, Aurora, Ontario L4G 6W6 (CA); ANTCZAK, Casimir, Gregory, Culver, IN 46511 (US); MORTIMER, Richard, Donald, Toronto, Ontario M4G 2V9 (CA); LU, Yee-Fung, Markham, Ontario L6E 1C5 (CA); HOCHFELLNER, Brian, George, Newmarket, Ontario L3X 2X8 (CA); ARSENAULT, Edward, Armand, Bradford, Ontario L3Z 3C2 (CA); YEH, Wen-Lung, Thornhill, Ontario L4J 7H6 (CA)
(74) Representative: Hoffmann Eitle
(86) International application number: PCT/US2003/006071
(87) International publication number: WO 2003/074481

(56) References cited:
- WO-A-02/096868

## Description

### BACKGROUND OF THE INVENTION

### Cross Reference to Related Applications

This application claims priority under 35 U.S.C. §119(e)(1) to provisional application number 60/360,847 which was filed on March 1, 2002.

### 1. Field of the Invention

This invention relates to the synthesis of prostamides, which may be useful as pharmaceutical compounds, e.g. as medicinal compounds useful for treating glaucoma and/or lower elevated intraocular pressure.

### 2. Description of Related Art

See Journal of Medicinal Chemistry, 1979, Vol. 22, No. 11, pp. 1346 et seq. and U.S. Patent 3,954,741 to Schaaf et al.

See U.S. Patent 4,195,183 to Gandolfi et al.

See PCT Patent WO 01/55101 A2.

See Eur. J. of Pharmacology 432(2001) 211-13.

See U.S. Patent 4,404,372 to Johnson et al.

### BRIEF SUMMARY OF THE INVENTION

The present invention provides a process for the preparation of a prostamide which comprises the steps of
(a) reacting first intermediate having the general formula I 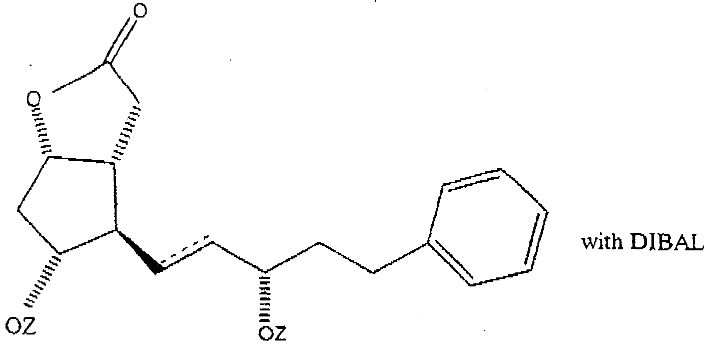 wherein Z is a protecting group and the dotted line represents the presence or absence of a double bond to yield a second intermediate having the general Formula II 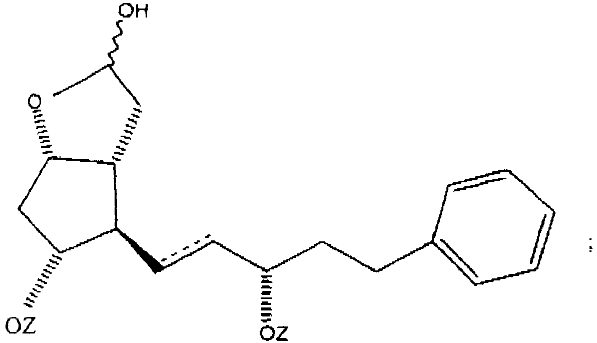
(b) reacting said second intermediate with
   Na(TMS)₂N and (C₆H₅)₃P⁺(CH₂)₄CONR₂X⁻
   wherein R is a C₁₋₅ alkyl or hydrogen and X⁻ is an anion to yield a third intermediate having the general formula III 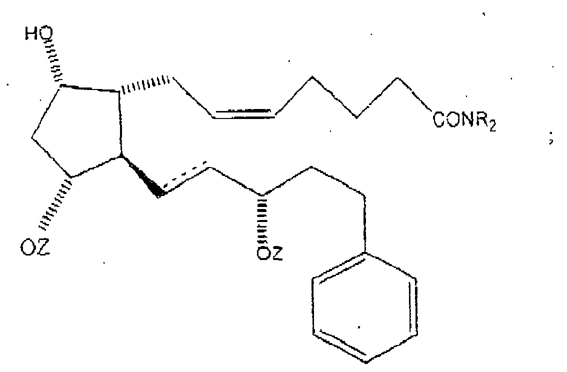
(c) reacting said third intermediate with a protecting agent to yield a fourth intermediate having the general formula IV 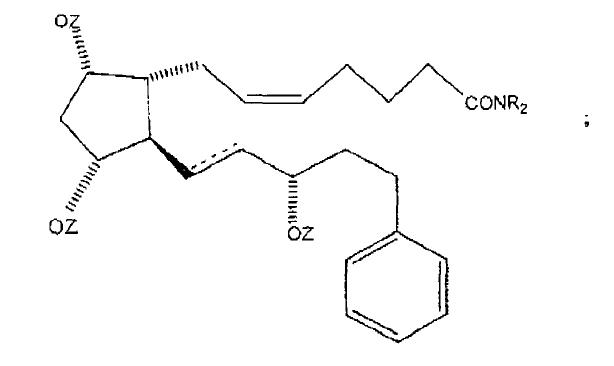
(d) sequentially reacting said fourth intermediate with DMSO and aqueous LiCl and removing said protecting groups to yield a prostamide having the general formula V

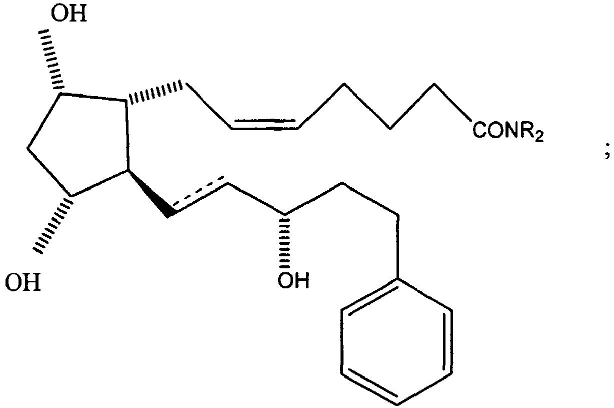

### DETAILED DESCRIPTION OF THE INVENTION

Preferably the present invention provides a process for the preparation of a prostamide which comprises the steps of
(b) reacting first intermediate having the general formula I 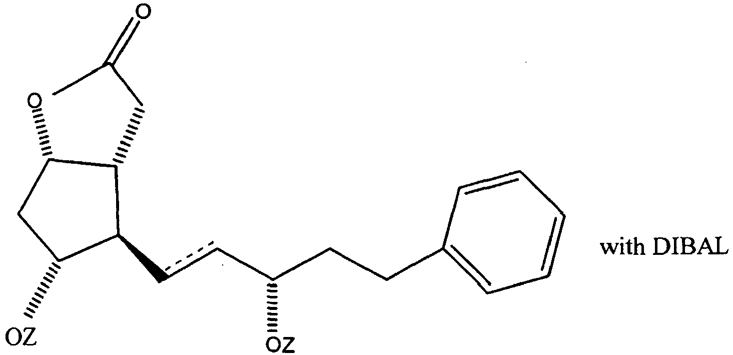 wherein Z is a protecting group and the dotted line represents the presence or absence of a double bond to yield a second intermediate having the general Formula II 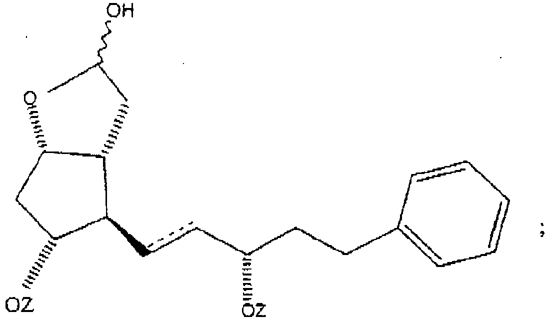
(b) reacting said second intermediate with
   Na(TMS)₂N and (C₆H₅)₃P⁺(CH₂)₄-CONR₂X⁻
   wherein R is a C₁₋₅ alkyl or hydrogen and X⁻ is an anion to yield a third intermediate having the general formula III 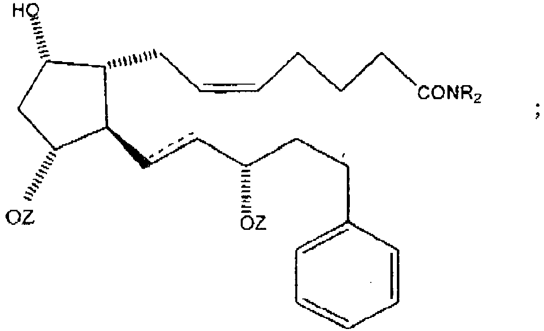
(c) reacting said third intermediate with TMSCl to yield a fourth intermediate having the general formula IV 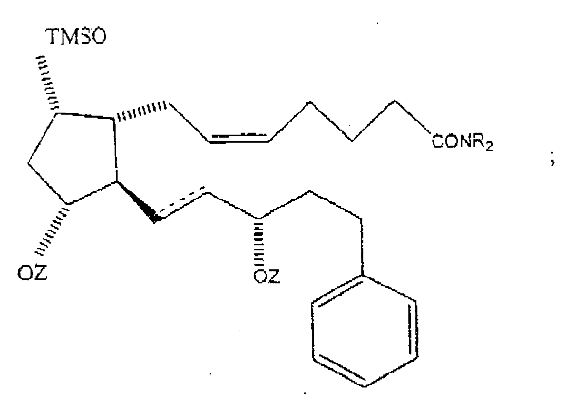
(d) sequentially reacting said fourth intermediate with DMSO and aqueous LiCl and removing said protecting groups to yield a prostamide having the general formula V

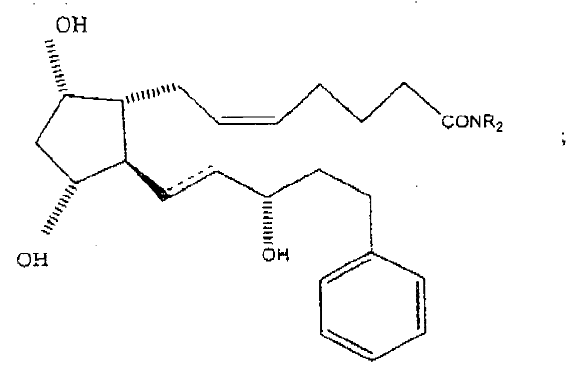

More preferably at least one R is hydrogen and the other is ethyl.

More preferably Z is TMS.

DIBAL is disobutylaluminum hydride.

TMS is trimethylsilyl.

### DESCRIPTION OF THE PREFERRED EMBODIMENT

This step of the synthesis produces the crude bimatoprost API by introduction of the upper side chain. The lactone moiety is first reduced to the lactol under standard diisobutylaluminum hydride conditions. The lactol is not isolated, but is treated directly in situ with Wittig reagent. The crude bimatoprost is a mixture of cis and trans isomers about the 5,6-double bond. The cis isomer is the major project. The trans isomer is controlled in the crude bimatoprost specifications by a limit of NMT 8% on each individual impurity.

## Claims

1. A process for the preparation of a prostamide which comprises the steps of
(a) reacting first intermediate having the general formula I 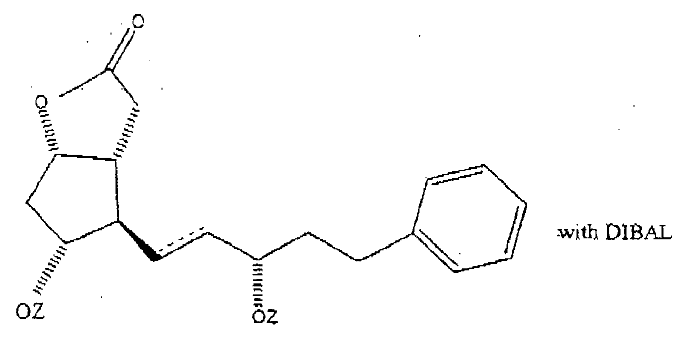 wherein Z is a protecting group and the dotted line represents the presence or
absence of a double bond to yield a second intermediate having the general Formula II 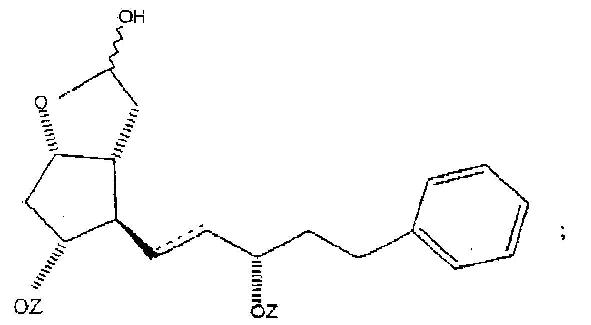
(b) reacting said second intermediate with
Na(TMS)₂N and (C₆H₅)₃P⁺(CH₂)₄CONR₂X⁻
wherein R is a C₁₋₃ alkyl or hydrogen and X⁻ is an anion to yield a third intermediate having the general formula III 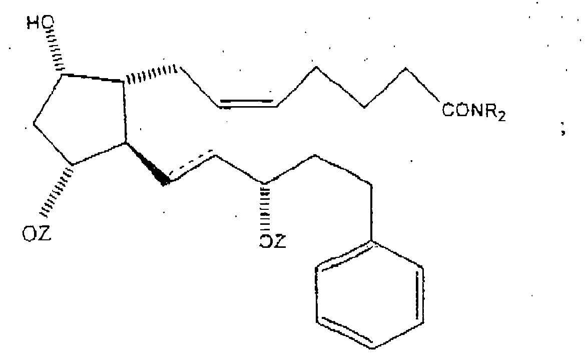
(c) reacting said third intermediate with a protecting agent TMSCl to yield a fourth intermediate having the general formula IV 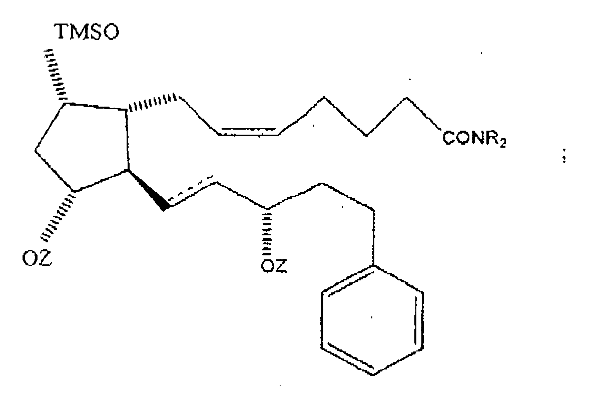
(d) sequentially reacting said fourth intermediate with DMSO and aqueous LiCl and removing said protecting groups to yield a prostamide having the general formula V

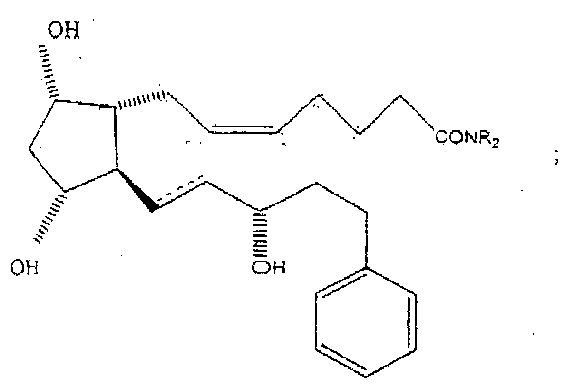

2. The process of claim 1 wherein one R is hydrogen and the other R is ethyl.

3. The process of claim 1 wherein Z is TMS.

## Patentansprüche

1. Verfahren zur Herstellung eines Prostamids, umfassend die Stufen:
(a) Umsetzen eines ersten Intermediats der allgemeinen Formel (I): 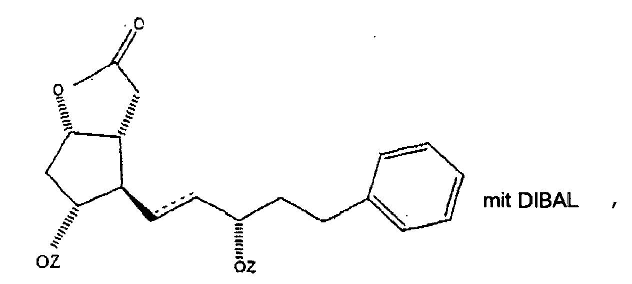 worin Z eine Schutzgruppe ist und die gestrichelte Linie die Gegenwart oder Abwesenheit einer Doppelbindung bezeichnet, um ein zweites Intermediat der allgemeinen Formel (II) zu ergeben: 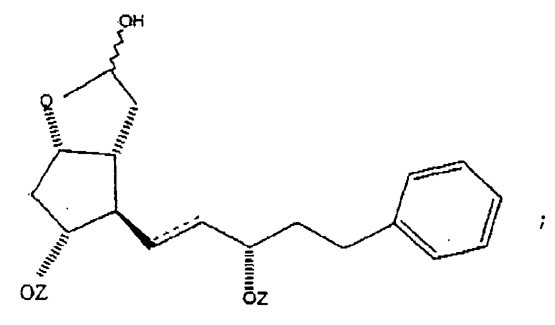
(b) Umsetzen des zweiten Intermediats mit Na(TMS)₂N und (C₆H₅)₃P⁺(CH₂)₄CONR₂X⁻ worin R C₁₋₅-Alkyl oder Wasserstoff ist und X⁻ Amino ist, um ein drittes Intermediat der allgemeinen Formel (III) zu ergeben: 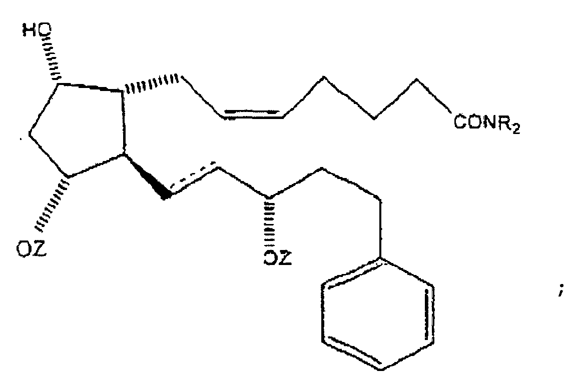
(c) Umsetzen des dritten Intermediats mit dem Schutzmittel TMSCl, um ein viertes Intermediat der allgemeinen Formel (IV) zu ergeben: 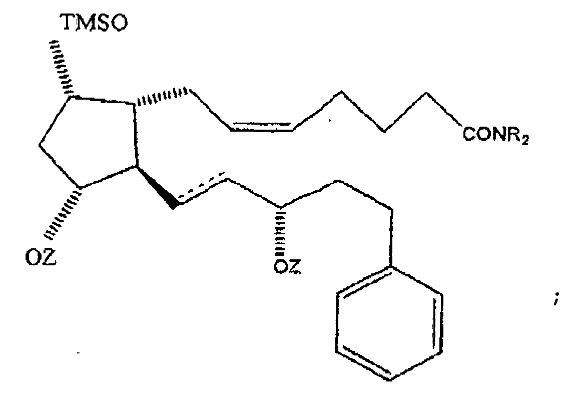
(d) aufeinanderfolgendes Umsetzen des vierten Intermediats mit DMSO und wässrigem LiCl und Entfernen der Schutzgruppen, um ein Prostamid der allgemeinen Formel (V) zu ergeben:

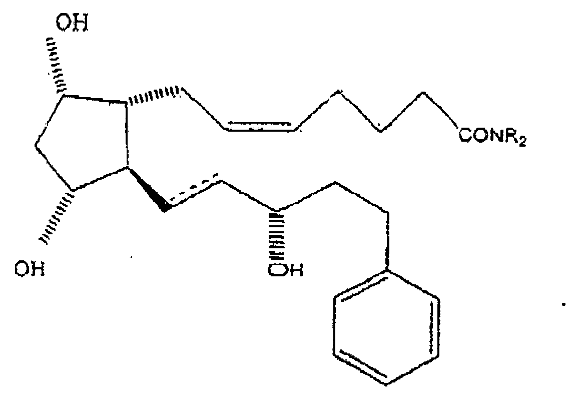

2. Verfahren gemäss Anspruch 1, worin ein R Wasserstoff und das andere R Ethyl ist.

3. Verfahren gemäss Anspruch 1, worin Z TMS ist.

## Revendications

1. Procédé pour la préparation d'un prostamide qui comprend les étapes de
(a) réaction d'un premier intermédiaire ayant la formule générale I 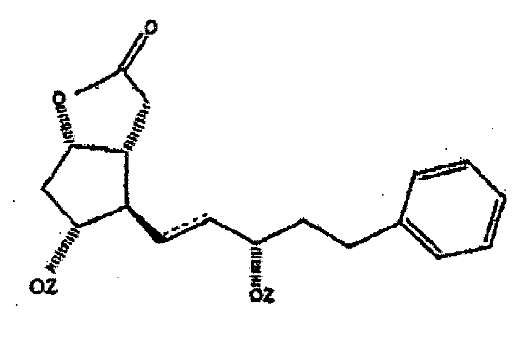 avec du DIBAL
dans laquelle Z est un groupe de protection et la ligne pointillée représente la présence ou l'absence d'une liaison double pour donner un deuxième intermédiaire ayant la formule générale II 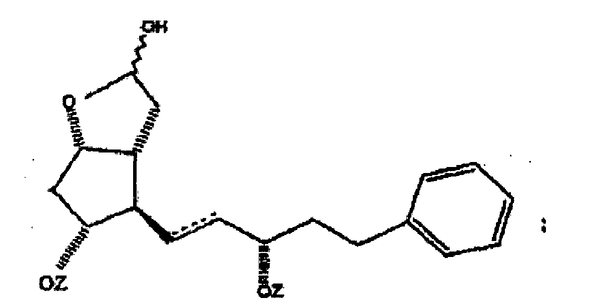
(b) réaction dudit deuxième intermédiaire avec
Na(TMS)₂N et (C₆H₅)₃P⁺(CH₂)₄CONR₂X⁻ dans laquelle R est un alkyle en C₁-C₅ ou un hydrogène et X⁻ est un anion pour donner un troisième intermédiaire ayant la formule générale III 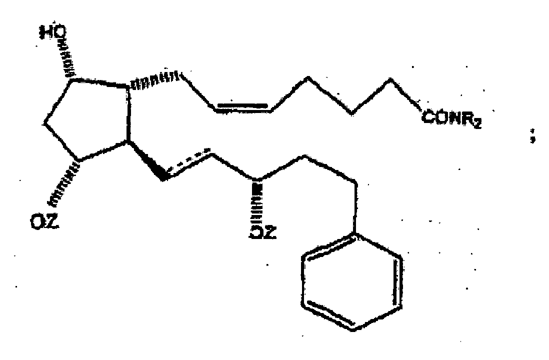
(c) réaction dudit troisième intermédiaire avec un agent de protection TMSCl pour donner un quatrième intermédiaire ayant la formule générale IV 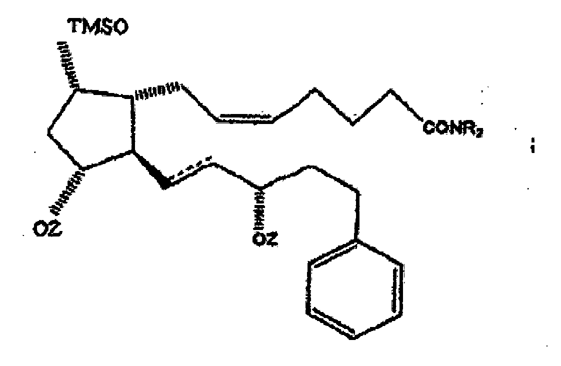
(d) réaction séquentielle dudit quatrième intermédiaire avec du DMSO et du LiCl aqueux et élimination desdits groupes de protection pour donner un prostamide ayant la formule générale V

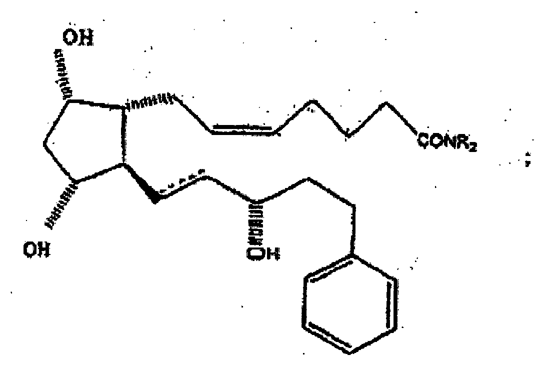

2. Procédé selon la revendication 1 dans lequel un R est un hydrogène et l'autre R est un éthyle.

3. Procédé selon la revendication 1 dans lequel Z est un TMS.
